# NOUVEAU FASCICULE DE BREVET EUROPEEN

(11) **EP 1 707 183 B2**
(45) Date de publication et mention de la décision concernant l'opposition: **25.04.2018**
(45) Mention de la délivrance du brevet: 03.03.2010
(21) Numéro de dépôt: 06111859.2
(22) Date de dépôt: 28.03.2006
(51) Int. Cl.: A61K 8/34, A61K 8/37, A61K 8/73, A61K 8/81, A61K 8/86, A61K 8/87, A61Q 5/06, A61Q 5/10

(54) **Composition colorante comprenant un polymère associatif non ionique, procédé de coloration de fibres kératiniques la mettant en oeuvre**
Färbemittel enthaltend ein nonionisches assoziatives Polymer und Färbeverfahren dafür
Dye composition comprising a non-ionic associative polymer, process for dyeing keratin fibres using same

(30) Priorité: 31.03.2005 FR 0550841
(43) Date de publication de la demande: 04.10.2006
(73) Titulaire: L'Oréal, 75008 Paris (FR)
(72) Inventeur: Legrand, Frédéric, Shinjuku-ku, 162-0842 Tokyo (JP)
(74) Mandataire: Fevrier, Murielle Françoise E.

(56) Documents cités:
- DE-A1- 3 030 119
- DE-A1- 3 834 142
- FR-A- 2 803 195
- US-A- 5 480 459
- US-A1- 2004 098 815
- US-B1- 6 800 098

## Description

La présente invention a pour objet une composition colorante comprenant au moins un colorant direct, au moins un alcool gras, au moins un ester d'acide gras, au moins un tensioactif non ionique et/ou anionique, au moins un polymère associatif non ionique ; la teneur en eau dans la composition colorante étant d'au moins 55% en poids.

Elle concerne de même un procédé de coloration de fibres kératiniques, notamment humaines, mettant en oeuvre une telle composition ainsi qu'un dispositif à plusieurs compartiments comprenant d'une part la composition colorante et d'autre part une composition oxydante.

Il existe essentiellement deux types de coloration des fibres kératiniques, notamment humaines, telles que les cheveux.

La première, appelée coloration d'oxydation ou encore coloration permanente, consiste à mettre en oeuvre des précurseurs de colorants d'oxydation, qui sont des substances peu ou non colorées. Lorsqu'ils sont mis en présence d'un agent oxydant, ces composés produisent, par un processus de condensation oxydative ayant lieu à l'intérieur même de la fibre, des substances colorées qui restent piégées dans la fibre.

La seconde, appelée coloration directe ou encore semi-permanente, est obtenue en mettant en oeuvre des composés colorés et colorants ayant une affinité avec les fibres kératiniques sur lesquelles ils sont appliqués. Ce type de coloration ne nécessite pas de mettre en oeuvre un agent oxydant pour révéler la couleur, bien qu'il ne soit pas exclu que ce type d'agent soit présent pendant le procédé. On parle alors dans ce dernier cas, de coloration directe éclaircissante.

Les compositions colorantes de l'état de la technique se présentent dans la majorité des cas, sous la forme de liquides, de gels ou de crèmes qui sont, si nécessaire, mélangés avant l'application sur les fibres, à une composition oxydante.

Les compositions colorantes sont le plus souvent relativement riches en matières premières, parmi lesquelles on trouve habituellement des corps gras, des tensioactifs et/ou des polymères. Ces compositions sont formulées de telle sorte qu'elles présentent des propriétés d'étalement et des textures faciles à travailler afin de permettre une application aisée et rapide sur les fibres, tout en étant suffisamment épaisses pour ne pas couler hors des zones que l'on souhaite colorer. De plus ces compositions doivent rester stables pendant le temps de pause sur les fibres et être facilement éliminables au rinçage une fois la coloration obtenue.

Or il n'est pas rare de constater que des quantités importantes de matières premières pénalisent les performances colorantes de telles compositions. On peut ainsi observer une cinétique moins favorable, une intensité amoindrie de la nuance obtenue, une moins bonne homogénéité de la couleur d'une fibre à l'autre et/ou selon l'endroit de la fibre (racine/pointe), etc.

La présente invention a donc pour objet de proposer des compositions colorantes, qui ne présentent pas les inconvénients ci-dessus mentionnés des compositions colorantes actuelles, tout en conservant les propriétés citées ci-dessus.

Un tel objectif est atteint par la présente invention qui a donc pour objet des compositions colorantes comprenant, dans un milieu approprié pour la teinture des fibres kératiniques :
- au moins un alcool gras ;
- au moins un ester d'acide gras et d'alcool en C₁-C₁₀ ; qui est un mono ester d'acide carboxylique, linéaire ou ramifié, saturé ou insaturé, en C₈-C₃₀ et d'un alcool mono hydroxylé, linéaire ou ramifié, saturé ou insaturé, en C₁-C₁₀;
- au moins un tensioactif non ionique, anionique, ou leurs mélanges ;
- au moins un polymère associatif non ionique
- ladite composition colorante contenant au moins 55 % d'eau.

L'invention a de plus pour objet un procédé de coloration de fibres kératiniques mettant en oeuvre une telle composition, le cas échéant en présence d'une composition oxydante.

Elle concerne enfin un dispositif comprenant un premier compartiment renfermant une composition colorante selon l'invention et comprenant un second compartiment renfermant une composition oxydante.

La composition selon l'invention occasionne moins de dégradation des propriétés de coloration et permet d'obtenir des colorations plus puissantes, plus homogènes et plus chromatiques, tout en conférant aux fibres traitées de bonnes propriétés cosmétiques et en limitant leur dégradation.

Les compositions conformes à la présente invention présentent par ailleurs une texture idéale pour un usage en teinture des fibres kératiniques humaines, et en particulier des cheveux. Elles sont en effet onctueuses, suffisamment épaisses pour une application rapide et facile, avec une bonne élimination au rinçage, sans pour autant couler hors des zones de la chevelure que l'on souhaite traiter.

Mais d'autres caractéristiques et avantages de l'invention apparaîtront plus clairement à la lecture de la description et des exemples ci-dessous.

Dans ce qui va suivre, et à moins d'une indication différente, il est précisé que les bornes de gammes de valeurs sont incluses dans ces gammes.

Lorsqu'il sera fait mention d'un composé à chaîne grasse, cette chaîne est une chaîne hydrocarbonée, saturée ou non, linéaire ou ramifiée, comprenant de 8 à 30 atomes de carbone, de préférence de 10 à 24 atomes de carbone.

De plus la présente invention est appropriée pour la coloration des fibres kératiniques, notamment humaines, telles que les cheveux plus particulièrement.

Ainsi, comme cela a été indiqué précédemment, la composition colorante selon l'invention possède une teneur en eau d'au moins 55 % en poids par rapport au poids de ladite composition colorante.

Conformément à un mode de réalisation préféré de l'invention, la teneur en eau représente au moins 60 % en poids par rapport au poids de ladite composition colorante.

La composition comprend par ailleurs au moins un alcool gras.

Cet alcool gras est non oxyalkyléné et non glycérolé.

L'alcool gras peut être choisi notamment parmi les alcools linéaires ou ramifiés, saturés ou insaturés, en C₈-C₃₀, plus particulièrement en C₁₀-C₂₄, de préférence C₁₂-C₂₄, comportant éventuellement au moins un autre groupement hydroxyle. A titre d'exemples, on peut citer entre autres les alcools oléique, laurique, palmitique, myristique, béhénique, stéarique, linoléique, linolénique, caprique, arachidonique, ou leurs mélanges.

Selon un mode de réalisation avantageux de l'invention, la teneur totale en alcools gras représente de 0,1 % à 30 % en poids de la composition colorante. De préférence, la teneur totale en alcools gras représente de 0,5 à 20 % en poids par rapport au poids de la composition colorante.

Avantageusement, la composition selon l'invention peut comprendre un autre corps gras différent des alcools gras précités. Ainsi, la composition peut comprendre à titre de corps gras au moins un composé choisi parmi les amides d'acide gras non oxyalkylénés et non glycérolés, les huiles minérales, les huiles végétales, ou leurs mélanges.

Les amides d'acide gras sont plus particulièrement choisis parmi les composés dérivant d'une alcanolamine et d'un acide gras en C₈-C₃₀. De préférence, ils sont choisis parmi les amides d'une alcanolamine en C₂-C₁₀ et d'un acide gras en C₁₄-C₃₀, et encore plus préférentiellement parmi les amides d'une alcanolamine en C₂-C₁₀ et d'un acide gras en C₁₄-C₂₂.

Avantageusement, l'amide d'acide gras est choisi parmi:
- le diéthanolamide d'acide oléique, tel que l'amide commercialisé sous la dénomination commerciale MEXANYL® GT par la société CHIMEX,
- le monoéthanolamide d'acide myristique, tel que l'amide commercialisé sous la dénomination commerciale COMPERLAN® MM par la société COGNIS,
- le diéthanolamide d'acides gras de soja, tel que l'amide commercialisé sous la dénomination commerciale COMPERLAN® VOD par la société COGNIS,
- l'éthanolamide d'acide stéarique, tel que l'amide commercialisé sous la dénomination commerciale MONAMID® S par la société UNIQEMA,
- le monoisopropanolamide d'acide oléique, tel que l'amide commercialisé sous la dénomination commerciale WITCAMIDE® 61 par la société WITCO,
- le diéthanolamide d'acide linoléique, tel que l'amide commercialisé sous la dénomination commerciale PURTON® SFD par la société ZSCHIMMER SCHWARZ,
- le monoéthanolamide d'acide stéarique, tel que l'amide commercialisé sous la dénomination commerciale MONAMID® 972 par la société ICI/UNIQEMA,
- le monoéthanolamide d'acide béhénique, tel que l'amide commercialisée sous la dénomination commerciale INCROMIDE® BEM de CRODA,
- le monoisopropanolamide d'acide isostéarique, tel que l'amide commercialisé sous la dénomination commerciale WITCAMIDE® SPA par la société WITCO,
- le diéthanolamide d'acide érucique, tel que l'amide commercialisé sous la dénomination commerciale diéthanolamide d'acide érucique par la société STEARINERIES DUBOIS,
- le monoéthanolamide d'acide ricinoléique, tel que l'amide commercialisé sous la dénomination commerciale monoéthanolamide ricinoléique par la société STEARINERIES DUBOIS.

L'huile de paraffine est un exemple d'huile minérale susceptible d'être utilisée comme corps gras dans la composition.

En ce qui concerne les huiles végétales, on peut citer tout particulièrement l'huile d'avocat, l'huile d'olive ou la cire liquide de jojoba.

Selon un mode de réalisation avantageux de l'invention, la teneur totale en corps gras différents des alcools gras précités représente de 0,1 % à 30 % en poids de la composition colorante. De préférence, la teneur totale en corps gras différents des alcools gras précités représente de 0,5 à 20 % en poids par rapport au poids de la composition colorante.

La composition selon l'invention comprend par ailleurs au moins un ester d'acide gras et d'alcool en C₁-C₁₀.
ledit ester est un mono-, ester d'acide carboxylique, linéaire ou ramifié, saturé ou insaturé, en C₈-C₃₀ et d'un alcool mono hydroxylé, linéaire ou ramifié, saturé ou insaturé, en C₁-C₁₀.

De préférence, l'ester est choisi parmi les mono- ester des acides oléique, laurique, palmitique, myristique, béhénique, stéarique, linoléique, linolénique, caprique, arachidonique, et de méthanol, éthanol, propanol, isopropanol, éthylèneglycol, glycérol, octanol, décanol, ainsi que leurs mélanges.

Selon un mode de réalisation particulier de l'invention, la teneur en ester d'acide gras et d'alcool en C₁-C₁₀ représente de 0,1 % à 30 % en poids par rapport au poids de la composition colorante. De préférence, la teneur en ester représente 0,5 à 20 % en poids par rapport au poids de la composition colorante. Selon une variante encore plus avantageuse, la teneur en ester représente 1 à 15 % en poids par rapport au poids de la composition colorante.

La composition selon l'invention comprend par ailleurs au moins un tensioactif non ionique, anionique, ou leurs mélanges.

En ce qui concerne les tensioactifs non ioniques, ces derniers sont plus particulièrement choisis parmi les tensioactifs non ioniques alcoxylés ou glycérolés, ou leurs mélanges.

De préférence, les tensioactifs non ioniques sont choisis parmi:
- les alcools gras oxyalkylénés ou glycérolés;
- les alkylphénols dont la chaîne alkyle est en C₈-C₁₈, oxyalkylénés ;
- les amides gras oxyalkylénés ou glycérolés ;
- les huiles végétales oxyalkylénées ;
- les esters d'acides C₆-C₃₀ du sorbitan, éventuellement oxyalkylénés ;
- les esters d'acides gras du sucrose, éventuellement oxyalkylénés ;
- les esters d'acides gras du polyéthylèneglycol ;
- les alkyl(C₆-C₃₀)polyglycosides ;
- les dérivés de N-alkyl(C₆-C₃₀)glucamine ;
- les oxydes d'amines tels que les oxydes d'alkyl (C₁₀ - C₁₄) amines ou les oxydes de N-acylaminopropylmorpholine ;
- les copolymères d'oxyde d'éthylène et de propylène ;
- leurs mélanges.

Plus particulièrement, le nombre moyen de motifs oxyalkylénés est avantageusement compris entre 2 et 150 motifs. De préférence, il s'agit de motifs oxyéthylénés, oxypropylénés ou leurs mélanges.

En ce qui concerne les tensioactifs glycérolés, ils comportent de préférence en moyenne 1 à 20 groupements glycérol et en particulier 1,5 à 5.

Conformément à un mode de réalisation particulièrement avantageux de l'invention, les tensioactifs non ioniques sont choisis parmi les alcools gras oxyalkylénés ou glycérolés.

Pour ce qui a trait aux tensioactifs anioniques, ces derniers sont habituellement choisis parmi :
- les alkyl(C₆-C₃₀)sulfates, les alkyl(C₆-C₃₀)éthersulfates, les alkyl(C₆-C₃₀)amido-éthersulfates, les alkylarylpolyéthersulfates, les monoglycérides sulfates ;
- les alkyl(C₆-C₃₀)sulfonates, les alkyl(C₆-C₃₀)amidesulfonates, les alkyl(C₆-C₃₀)aryl-sulfonates, les α-oléfine-sulfonates, les paraffine-sulfonates ;
- les alkyl(C₆-C₃₀)phosphates;
- les alkyl(C₆-C₃₀)sulfosuccinates, les alkyl(C₆-C₃₀) éthersulfosuccinates, les alkyl(C₆-C₃₀) amidesulfosuccinates;
- les alkyl(C₆-C₃₀)sulfoacétates;
- les acyl(C₆-C₂₄)sarcosinates ;
- les acyl(C₆-C₂₄)glutamates;
- les éthers d'alkyl(C₆-C₃₀)polyglycosides carboxyliques ;
- les alkyl(C₆-C₃₀) polyglycoside sulfosuccinates ;
- les alkyl(C₆-C₃₀)sulfosuccinamates ;
- les acyl(C₆-C₂₄)iséthionates ;
- les N-acyl(C₆-C₂₄)taurates ;
- les sels d'acides gras ;
- les acyl(C₈-C₂₀)-lactylates ;
- les sels d'acides d'alkyl(C₆-C₃₀)-D galactoside uroniques ;
- les sels d'acides alkyl(C₆-C₃₀)éther carboxyliques polyoxyalkylénés, d'alkyl(C₆-C₃₀)aryl éther carboxyliques polyoxyalkylénés, d'alkyl(C₆-C₃₀)amido éther carboxyliques polyoxyalkylénés;
- et leurs mélanges.

Ces tensioactifs anioniques se trouvent avantageusement sous forme de sels dans la composition selon l'invention, notamment de sels de métaux alcalins, comme le sodium ; de métaux alcalino-terreux, comme par exemple le magnésium ; de sels d'ammonium ; de sels d'amines; de sels d'aminoalcools. Ils pourraient aussi, selon les conditions, se trouver sous leur forme acide.

A noter que les radicaux alkyle ou acyle de ces différents composés comportent de préférence de 12 à 20 atomes de carbone. De préférence, le radical aryle désigne un groupement phényle ou benzyle.

De plus, les tensioactifs anioniques polyoxyalkylénés comportent de préférence de 2 à 50 groupements oxyde d'alkylène en particulier d'éthylène.

Avantageusement, la teneur en tensioactif(s) non ionique(s), anionique(s) et leurs mélange, représente de 0,1% à 40 % en poids par rapport au poids de la composition colorante, plus particulièrement de 0,5 à 30 % en poids par rapport au poids de la composition colorante, de préférence de 1 à 20 % en poids par rapport au poids de la composition colorante.

La composition comprend par ailleurs au moins un polymère associatif non ionique.

Plus particulièrement, les polymères associatifs sont choisis parmi :
- **(1)** les celluloses modifiées par des groupements comportant au moins une chaîne grasse ;
   pour lesquelles peut citer à titre d'exemples:
   ∘ les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS® (alkyles en C₁₆) vendu par la société AQUALON, ou le produit BERMOCOLL EHM 100® vendu par la société BEROL NOBEL,
   ∘ celles modifiées par des groupes polyalkylène glycol éther d'alkyl phénol, tel que le produit AMERCELL POLYMER HM-1500® (polyéthylène glycol (15) éther de nonyl phénol) vendu par la société AMERCHOL.
- **(2)** les hydroxypropylguars modifiés par des groupements comportant au moins une chaîne grasse, tel que par exemple, le produit ESAFLOR HM 22® (chaîne alkyle en C₂₂) vendu par la société LAMBERTI, les produits RE210-18® (chaîne alkyle en C₁₄) et RE205-1® (chaîne alkyle en C₂₀) vendus par la société RHONE POULENC.
- **(3)** les copolymères de vinyl pyrrolidone et de monomères hydrophobes à chaîne grasse dont on peut citer à titre d'exemples :
   ∘ les produits ANTARON V216® ou GANEX V216® (copolymère vinylpyrrolidone / hexadécène) vendu par la société I.S.P.
   ∘ les produits ANTARON V220® ou GANEX V220® (copolymère - vinylpyrrolidone / eicosène) vendu par la société I.S.P.
- **(4)** les copolymères de méthacrylates ou d'acrylates d'alkyles en C₁-C₆ et de monomères amphiphiles comportant au moins une chaîne grasse, tel que par exemple le copolymère acrylate de méthyle/acrylate de stéaryle oxyéthyléné vendu par la société GOLDSCHMIDT sous la dénomination ANTIL 208®.
- **(5)** les copolymères de méthacrylates ou d'acrylates hydrophiles et de monomères hydrophobes comportant au moins une chaîne grasse tel que par exemple le copolymère méthacrylate de polyéthylèneglycol/méthacrylate de lauryle.
- **(6)** les polyuréthanes polyéthers comportant dans leur chaîne, à la fois des séquences hydrophiles, de nature le plus souvent polyoxyéthylénée, et des séquences hydrophobes qui peuvent être par exemple des enchaînements aliphatiques seuls et/ou des enchaînements cycloaliphatiques et/ou aromatiques.
- **(7)** les polymères à squelette aminoplaste éther possédant au moins une chaîne grasse, tels que les composés PURE THIX® proposés par la société SUD-CHEMIE.

De préférence, les polyéthers polyuréthanes comportent au moins deux chaînes lipophiles hydrocarbonées, ayant de 8 à 30 atomes de carbone, séparées par une séquence hydrophile, les chaînes hydrocarbonées pouvant être des chaînes pendantes ou des chaînes en bout de séquence hydrophile.

En particulier, il est possible qu'une ou plusieurs chaînes pendantes soient prévues.

En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non ioniques comportent une liaison uréthanne entre les séquences hydrophiles, d'où l'origine du nom.

Par extension figurent aussi parmi les polyéthers polyuréthanes non ioniques à chaîne grasse, ceux dont les séquences hydrophiles sont liées aux séquences lipophiles par d'autres liaisons chimiques.

A titre d'exemples de polyéthers polyuréthanes non ioniques à chaîne grasse utilisables dans l'invention, on peut aussi utiliser aussi le Rhéolate 205® à fonction urée vendu par la société RHEOX ou encore les Rhéolates® 208 , 204 ou 212, ainsi que l'Acrysol RM 184®.

On peut également citer le produit ELFACOS T210® à chaîne alkyle en C₁₂-C₁₄ et le produit ELFACOS T212® à chaîne alkyle en C₁₈ de chez AKZO.

Le produit DW 1206B® de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Plus particulièrement encore, selon l'invention, on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations Aculyn 46® et Aculyn 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

Avantageusement, la composition présente une teneur en polymère(s) associatif(s) non ionique(s) comprise entre 0,01 et 5 % en poids par rapport au poids de la composition colorante. De préférence, la teneur en polymère associatif est comprise entre 0,02 à 3% en poids par rapport au poids de la composition colorante.

La composition selon l'invention comprend en outre au moins un colorant direct.

Plus particulièrement, le ou les colorants directs sont de nature non ionique, cationique ou anionique.

A titre d'exemples non limitatifs, on peut citer les colorants benzéniques nitrés, les colorants azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane et les colorants naturels, seuls ou en mélanges.

Il peut par exemple être choisi parmi les colorants benzéniques nitrés rouges ou orangés suivants :
- le 1-hydroxy-3-nitro-4-N-(γ-hydroxypropyl)amino benzène,
- le N-(β-hydroxyéthyl)amino-3-nitro-4-amino benzène,
- le 1-amino-3-méthyt-4-N-(β-hydroxyéthyl)amino-6-nitro benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- le 1-amino-2-nitro-4-(β-hydroxyéthyl)amino-5-chloro benzène,
- la 2-nitro-4-amino-diphénylamine,
- le1-amino-3-nitro-6-hydroxybenzène.
- le 1-(β-aminoéthyl)amino-2-nitro-4-(β-hydroxyéthyloxy)benzène,
- le 1-(β, γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine,
- le 1-amino-2-nitro-4-hydroxy-5-méthylbenzène.

Le colorant direct peut aussi être choisi parmi les colorants directs benzéniques nitrés jaunes et jaune-verts, on peut par exemple citer les composés choisis parmi :
- le 1-β-hydroxyéthyloxy-3-méthylamino-4-nitrobenzène,
- le 1-méthylamino-2-nitro-5-(β,γ-dihydroxypropyl)oxy benzène,
- le 1-(β-hydroxyéthyl)amino-2-méthoxy-4-nitrobenzène,
- le 1-(β-aminoéthyl)amino-2-nitro-5-méthoxy-benzène,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyl)amino-3-nitro-méthylbenzène,
- le 4-(β,γ-dihydroxypropyl)amino-3-nitro-trifluorométhylbenzène,
- le 1-(β-uréidoéthyl)amino-4-nitrobenzène,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène,
- le 4-(β-hydroxyéthyl)amino-3-nitrobenzamide.

On peut aussi mentionner les colorants directs benzéniques nitrés bleus ou violets, comme par exemple :
- le 1-(β-hydroxyéthyl)amino-4-N,N-bis-(β-hydroxyéthyl)amino 2-nitrobenzène,
- le 1-(γ-hydroxypropyl)amino 4-N,N-bis-(β-hydroxyéthyl)amino 2-nitrobenzène,
- le 1-(β-hydroxyéthyl)amino 4-(N-méthyl, N-β-hydroxyéthyl)amino 2-nitrobenzène,
- le 1-(β-hydroxyéthyl)amino 4-(N-éthyl, N-β-hydroxyéthyl)amino 2-nitrobenzène,
- le 1-(β,γ-dihydroxypropyl)amino 4-(N-éthyl, N-β-hydroxyéthyl)amino 2-nitrobenzène,
- les 2-nitroparaphénylènediamines de formule suivante : 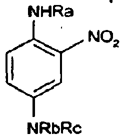 dans laquelle :
   - Rb représente un radical alkyle en C₁-C₄, un radical β-hydroxyéthyle ou β-hydroxypropyle ou γ-hydroxypropyle ;
   - Ra et Rc, identiques ou différents, représentent un radical β-hydroxyéthyle, -β-hydroxypropyle, γ-hydroxypropyle, ou β,γ-dihydroxypropyle, l'un au moins des radicaux Rb, Rc ou Ra représentant un radical γ-hydroxypropyle et Rb et Rc ne pouvant désigner simultanément un radical β-hydroxyéthyle lorsque Rb est un radical γ-hydroxypropyle, telles que celles décrits dans le brevet français FR 2 692 572.

Parmi les colorants directs azoïques utilisables selon l'invention on peut citer les colorants azoïques cationiques décrits dans les demandes de brevets WO 95/15144, WO 95/01772 et EP 714954, FR 2 822 696, FR 2 825 702, FR 2 825 625, FR 2 822 698, FR 2 822 693, FR 2 822 694, FR 2 829 926, FR 2 807 650, WO 02/078660, WO 02/100834, WO 02/100369, FR 2 844 269.

Parmi ces composés on peut tout particulièrement citer les colorants suivants:
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium

On peut également citer parmi les colorants directs azoïques les colorants suivants, décrits dans le COLOUR INDEX INTERNATIONAL 3e édition : Disperse Red 17, Acid Yellow 9, Acid Black 1, Basic Red 22, Basic Red 76, Basic Yellow 57, Basic Brown 16, Acid Yellow 36, Acid Orange 7, Acid Red 33, Acid Red 35, Basic Brown 17, Acid Yellow 23, Acid Orange 24, Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques on peut citer les colorants suivants : Disperse Red 15, Solvent Violet 13, Acid Violet 43, Disperse Violet 1, Disperse Violet 4, Disperse Blue 1, Disperse Violet 8, Disperse Blue 3, Disperse Red 11, Acid Blue 62, Disperse Blue 7, Basic Blue 22, Disperse Violet 15, Basic Blue 99, ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques on peut citer les composés suivants :
- Basic Blue 17, Basic Red 2.

Parmi les colorants directs méthiniques cationiques, on peut aussi citer le Basic Red 14, le Basic Yellow 13 et le Basic Yellow 29.

Parmi les colorants triarylméthaniques utilisables selon l'invention, on peut citer les composés suivants : Basic Green 1, Acid blue 9, Basic Violet 3, Basic Violet 14, Basic Blue 7, Acid Violet 49, Basic Blue 26, Acid Blue 7.

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

La composition peut aussi comprendre des colorants directs naturels comme la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

La teneur en colorants directs, représente avantageusement de 0,0005 à 15 % en poids par rapport au poids de la composition colorante, et de préférence de 0,005 à 12 % en poids par rapport au poids de la composition colorante. Selon un mode de réalisation encore plus avantageux de l'invention, la teneur en colorants directs, représente de 0,01 à 5 % en poids par rapport au poids de la composition colorante.

La composition selon l'invention peut comprendre en outre au moins un agent alcalinisant.

Parmi les agents alcalinisants on peut citer, à titre d'exemple, l'ammoniaque, les carbonates alcalins, les alcanolamines en C₂-C₁₀ telles que les mono-, di- et triéthanolamines ainsi que leurs dérivés, les hydroxyalkylamines et les ethylènediamines oxyéthylénées et/ou oxypropylénées, les hydroxydes de sodium ou de potassium, les silicates de métaux alcalins ou alcalino-terreux, et les composés de formule suivante : 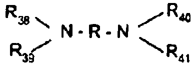 dans laquelle R est un reste propylène éventuellement substitué par un groupement hydroxyle ou un radical alkyle en C₁-C₄ ; R₃₈, R₃₉, R₄₀ et R₄₁, identiques ou différents, représentent un atome d'hydrogène, un radical alkyle en C₁-C₄ ou hydroxyalkyle en C₁-C₄.

De préférence, l'agent alcalinisant est choisi parmi l'ammoniaque, les alcanolamines, et les associations d'alcanolamines avec des silicates de métaux alcalins ou alcalino-terreux.

Selon un mode de réalisation avantageux de l'invention, la composition ne comprend pas d'ammoniaque en tant qu'agent alcalinisant.

A noter de plus que le pH peut aussi être ajusté en employant des agents acidifiants, comme par exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Plus particulièrement, la teneur en agent alcalinisant et/ou acidifiant est telle que le pH de la composition colorante est compris entre 3 et 12, avantageusement entre 4 et 11 et de préférence entre 7 et 11.

La composition selon l'invention peut aussi comprendre un ou plusieurs polymères substantifs cationiques ou amphotères.

Il est à noter qu'au sens de la présente invention, l'expression "polymère cationique" désigne tout polymère contenant des groupements cationiques et/ou des groupements ionisables en groupements cationiques.

De tels polymères peuvent être choisis parmi ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶, et de préférence comprise entre 10³ et 3.10⁶.

Parmi les polymères cationiques, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères, on peut citer :
**(1)** Les homopolymères ou copolymères dérivés d'esters ou d'amides acryliques ou méthacryliques et comportant au moins un des motifs de formules (I), (II), (III) ou (IV) suivantes: 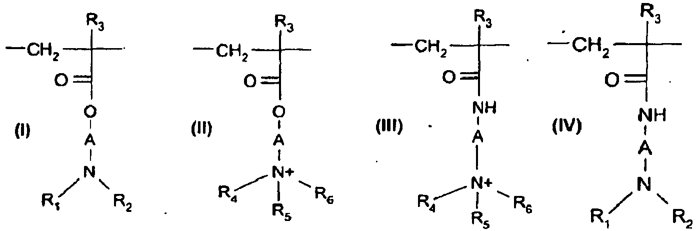 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.

   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques. Ainsi, parmi ces polymères de la famille (1), on peut citer :
   ∘ les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
   ∘ les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
   ∘ le copolymère d'acrylamide et de méthosulfate de méthacryloyloxyéthyltriméthylammonium vendu sous la dénomination RETEN par la société HERCULES,
   ∘ les copolymères vinylpyrrolidone / acrylate ou méthacrylate de dialkylaminoalkyle quaternisés ou non, tels que les produits vendus sous la dénomination "GAFOUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   ∘ les terpolymères méthacrylate de diméthyl amino éthyle/ vinylcaprolactame/ vinylpyrrolidone tel que le produit vendu sous la dénomination GAFFIX VC 713 par la société ISP,
   ∘ les copolymère vinylpyrrolidone / méthacrylamidopropyl dimethylamine commercialisés notamment sous la dénomination STYLEZE CC 10 par ISP,
   ∘ et les copolymères vinylpyrrolidone / méthacrylamide de diméthylaminopropyle quaternisés tel que le produit vendu sous la dénomination "GAFQUAT HS 100" par la société ISP.
**(2)** Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium .
**(3)** Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
**(4)** Les gommes de guar cationiques décrits plus particulièrement dans les brevets US 3589578 et US 4031307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
**(5)** Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
**(6)** Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2252840 et 2368508.
**(7)** Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1583363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
**(8)** Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3227615 et 2961347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
**(9)** Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 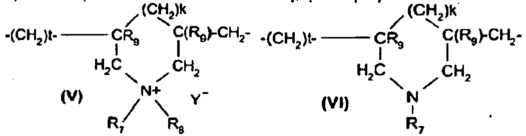 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y- est un anion tel que bromure, chlorure, acétate, borate, citrate, tartrate, bisulfate, bisulfite, sulfate, phosphate. Ces polymères sont notamment décrits dans le brevet français 2080759 et dans son certificat d'addition 2190406.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".
**(10)** Le polymère de diammonium quaternaire contenant des motifs récurrents répondant à la formule : 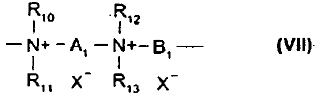 formule (VII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ;
   A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A₁, R₁₀ et R₁₂ peuvent former avec les deux atomes d'azote auxquels ils sont rattachés un cycle pipérazinique ; en outre si A₁ désigne un radical alkylène ou hydroxyalkylène linéaire ou ramifié, saturé ou insaturé, B1 peut également désigner un groupement -(CH₂)ₙ-CO-D-OC-(CH₂)ₙ- dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)ₓ-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH-.
         De préférence, X⁻ est un anion tel que le chlorure ou le bromure.
         Ces polymères ont une masse moléculaire moyenne en nombre généralement comprise entre 1000 et 100000.
         Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
         On peut utiliser plus particulièrement les polymères qui sont constitués de motifs récurrents répondant à la formule (VIII) suivante: 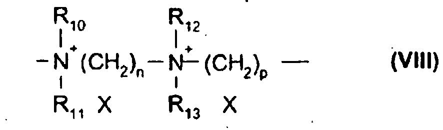 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
**(11)** Les polymères de polyammonium quaternaire constitués de motifs récurrents de formule (IX) : 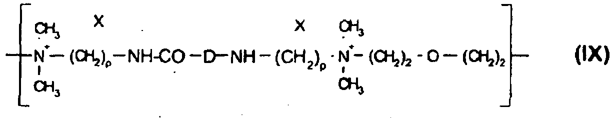 dans laquelle p désigne un nombre entier variant de 1 à 6 environ, D peut être nul ou peut représenter un groupement -(CH₂)ᵣ-CO- dans lequel r désigne un nombre égal à 4 ou à 7, X⁻ est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
   Parmi eux, on peut par exemple citer, les produits "Mirapol A 15", "Mirapol AD1", "Mirapol AZ1" et "Mirapol 175" vendus par la société Miranol.
**(12)** Les polymères quaternaires de vinylpyrrolidone et de vinylimidazole tels que par exemple les produits commercialisés sous les dénominations Luviquat FC 905, FC 550 et FC 370 par la société B.A.S.F.
**(13)** Les polyamines comme le Polyquart H vendu par HENKEL, référencé sous le nom de " POLYETHYLENEGLYCOL (15) TALLOW POLYAMINE "dans le dictionnaire CTFA.
**(14)** Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société ALLIED COLLOIDS.
**(15)** D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères aux motifs récurrents de formules (W) et (U) suivantes : 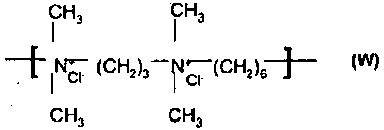 et notamment ceux dont le poids moléculaire, déterminé par chromatographie par perméation de gel, est compris entre 9500 et 9900; 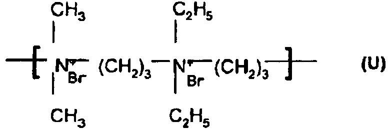 et notamment ceux dont le poids moléculaire, déterminé par chromatographie par perméation de gel, est d'environ 1200.

En ce qui concerne les polymères amphotères utilisables conformément à la présente invention, ceux-ci peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
**(1)** Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
   Le composé vinylique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société CALGON.
**(2)** Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butyl aminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
**(3)** Les polyaminoamides réticulés et alcoylés partiellement ou totalement dérivant de polyaminoamides de formule générale :

   -[-CO-R₁₉-CO-Z-]- **(X)**

   dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 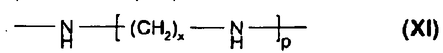 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (XI) ci-dessus, dans lequel x=2 et p=1 et qui dérive de l'éthylènediamine, ou le radical dérivant de la pipérazine : 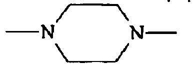
   c) dans les proportions de 0 à 20 moles % le radical -NH-(CH₂)₆-NH-dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.

   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.
**(4)** Les polymères comportant des motifs zwittérioniques de formule: 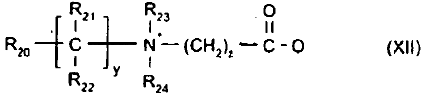 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de méthyle / diméthyl-carboxyméthylammonio-éthylméthacrylate de méthyle tel que le produit vendu sous la dénomination DIAFORMER Z301 par la société SANDOZ.
**(5)** Les polymères dérivés du chitosane comportant des motifs monomères répondant aux formules (XIII), (XIV), (XV) suivantes : 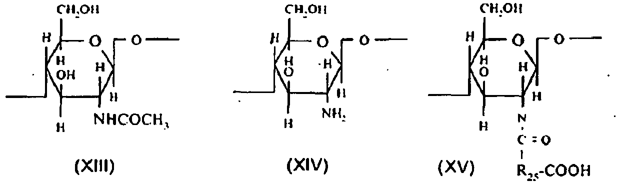 le motif (XIII) étant présent dans des proportions comprises entre 0 et 30%, le motif (XIV) dans des proportions comprises entre 5 et 50% et le motif F dans des proportions comprises entre 30 et 90%, étant entendu que dans ce motif (XV), R₂₅ représente un radical de formule : 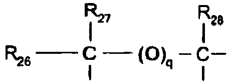 dans laquelle
   si q=0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₆ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
**(6)** Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
**(7)** Les polymères répondant à la formule générale (XVI) tels que ceux décrits par exemple dans le brevet français 1400366 : 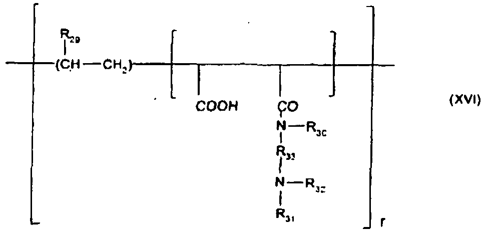
   dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁ désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)₂, R₃₃ représentant un groupement -CH₂-CH₂-, -CH₂-CH₂-CH₂-, -CH₂-CH(CH₃)-, R₃₁ ayant les significations mentionnées ci-dessus,
   ainsi que les homologues supérieurs de ces radicaux et contenant jusqu'à 6 atomes de carbone.
**(8)** Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- **(XVII)**

      où D désigne un radical 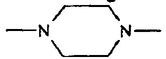 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne;
   b) les polymères de formule :

      -D-X-D-X- **(XVIII)**

      où D désigne un radical 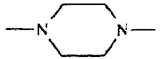 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
**(9)** Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1).

Selon l'invention, le ou les polymères substantifs cationiques ou amphotères, lorsqu'ils sont présents, représentent avantageusement de 0,01 % à 10 % en poids par rapport au poids de la composition colorante, plus particulièrement de 0,05 % à 5 % en poids par rapport au poids de la composition colorante, et de préférence de 0,1 % à 3 % en poids par rapport au poids de la composition colorante.

Le milieu approprié pour la teinture des fibres kératiniques est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions comprises entre 1 et 40 % en poids par rapport au poids de la composition colorante, et de préférence entre 5 et 30 % en poids par rapport au poids de la composition colorante.

La composition peut encore comprendre des additifs usuels dans le domaine comme notamment des agents épaississants organiques ou minéraux ; des agents antioxydants ; des agents de pénétration ; des agents séquestrants ; des parfums ; des tampons ; des agents dispersants ; des agents de conditionnement différents des polymères substantifs cationiques ou amphotères tels que par exemple des cations, des silicones volatiles ou non volatiles, modifiées ou non modifiées ; des agents filmogènes ; des céramides ; des agents conservateurs ; des agents stabilisants ; des agents opacifiants.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

Comme indiqué auparavant, la présente invention concerne de plus un procédé de coloration de matières kératiniques mettant en oeuvre la composition selon l'invention.

Selon une première variante, le procédé consiste à appliquer la composition en l'absence d'un agent oxydant, sur les matières kératiniques, de préférence des fibres, sèches ou humides, avec ou sans rinçage final de la composition.

Dans le cas de cette variante, la composition selon l'invention ne comprend pas de précurseur de colorant d'oxydation, mais seulement un ou plusieurs colorants directs.

Selon une deuxième variante de l'invention, le procédé consiste à appliquer la composition selon l'invention, en présence d'un agent oxydant, sur les matières kératiniques, sèches ou humides, puis à laisser poser pendant une durée suffisante pour obtenir la coloration souhaitée.

Selon une première possibilité, on applique sur lesdites fibres kératiniques, une ou plusieurs compositions colorantes selon l'invention et une composition oxydante simultanément ou successivement sans rinçage intermédiaire.

De préférence, la composition appliquée est une composition dite prête à l'emploi, c'est-à-dire une composition obtenue par mélange extemporané d'une ou de plusieurs compositions colorantes selon l'invention, avec une composition comprenant au moins un agent oxydant.

Dans ce cas un éclaircissement des fibres kératiniques est souhaité en association avec la coloration.

Bien évidemment la composition colorante peut comprendre une association de précurseurs de colorants d'oxydation et de colorants directs.

L'agent oxydant présents dans la composition oxydante peut être choisi par exemple parmi le peroxyde d'hydrogène, le peroxyde d'urée, les bromates de métaux alcalins, les persels tels que les perborates et persulfates, et les enzymes telles que les peroxydases et les oxydo-réductases à deux ou à quatre électrons. L'utilisation du peroxyde d'hydrogène est particulièrement préférée.

La teneur en agent oxydant est en général comprise entre 1 et 40 % en poids, par rapport au poids de la composition prête à l'emploi de préférence entre 1 et 20 % en poids par rapport au poids de la composition prête à l'emploi.

Généralement, la composition oxydante utilisée est une composition aqueuse et peut se trouver sous la forme d'une solution ou encore d'une émulsion.

Habituellement, on mélange la composition exempte d'agent oxydant avec environ 0,5 à 10 équivalents en poids de la composition oxydante.

Notons que le pH de la composition prête à l'emploi est plus particulièrement compris entre 3 et 12, de préférence entre 4 et 11 et encore plus précisément entre 6,5 et 10,5.

Le pH de la composition prête à l'emploi peut être ajusté au moyen d'un agent alcalinisant ou acidifiant notamment choisi parmi ceux mentionnés auparavant.

Toujours dans le cas où la composition est appliquée en présence d'un agent oxydant, le procédé peut comprendre une étape préliminaire consistant à stocker sous forme séparée, d'une part, une ou plusieurs compositions colorantes selon l'invention et d'autre part, une composition comprenant, dans un milieu approprié pour la teinture des fibres kératiniques humaines, au moins un agent oxydant, puis à procéder à leur mélange au moment de l'emploi avant d'appliquer ce mélange sur les matières kératiniques, sèches ou humides.

Quelle que soit la variante retenue, c'est-à-dire en présence ou en l'absence d'agent oxydant, le temps nécessaire au développement de la coloration est d'environ quelques secondes à 60 minutes et plus particulièrement d'environ 1 à 50 minutes.

La température nécessaire au développement de la coloration est généralement comprise entre la température ambiante (15 à 25°C) et 250°C, plus particulièrement entre la température ambiante et 180°c, de préférence entre la température ambiante et 60°C.

Une fois le temps nécessaire au développement de la coloration écoulé, on élimine de préférence la composition.

Ceci peut avoir lieu de manière classique, soit en mettant en oeuvre au moins un rinçage, soit en mettant en oeuvre un ou plusieurs lavages et rinçage(s), soit en mettant en oeuvre leur combinaison. Enfin, on sèche les matières kératiniques ou on les laisse sécher.

## Revendications

1. Composition colorante comprenant, dans un milieu approprié pour la teinture des fibres kératiniqués :
• au moins un colorant direct ;
• au moins un alcool gras ;
• au moins un ester d'acide gras et d'alcool en C₁-C₁₀ qui est un mono ester d'acide carboxylique, linéaire ou ramifié, saturé ou insaturé, en C₈-C₃₀ et d'un alcool mono hydroxylé, linéaire ou ramifié, saturé ou insaturé, en C₁-C₁₀;
• au moins un tensioactif non ionique, anionique ou leurs mélanges ;
• au moins un polymère associatif non ionique ;
• la teneur en eau étant d'au moins 55% en poids par rapport au poids de la composition colorante.

2. Composition selon la revendication précédente, **caractérisée en ce que** la teneur en eau représente au moins 60 % en poids par rapport au poids de ladite composition colorante.

3. Composition selon l'une quelconque des revendications précédentes, **caractérisée en ce que** la teneur en alcool gras représente de 0,1 à 30 % en poids par rapport au poids de la composition colorante.

4. Composition selon la revendication précédente, **caractérisée en ce que** l'ester est choisi parmi les mono esters des acides oléique, laurique, palmitique, myristique, béhénique, stéarique, linoléique, linolénique, caprique, arachidonique, et de méthanol, éthanol, propanol, isopropanol, éthylèneglycol, glycérol, octanol, décanol, ainsi que leurs mélanges.

5. Composition selon l'une quelconque des revendications précédentes, **caractérisée en ce que** la teneur en ester d'acide gras et d'alcool en C₁-C₁₀ est comprise entre 0,1 et 30 % en poids par rapport au poids de la composition colorante.

6. Composition selon l'une quelconque des revendications précédentes, **caractérisée en ce que** le tensioactif non ionique est choisi parmi :
• les alcools gras oxyalkylénés ou glycérolés ;
• les alkylphénols dont la chaîne alkyle est en C₈-C₁₈, oxyalkylénés ;
• les amides gras oxyalkylénés ou glycérolés ;
• les huiles végétales oxyalkylénées ;
• les esters d'acides gras du sorbitan, éventuellement oxyalkylénés ;
• les esters d'acides gras du sucrose, éventuellement oxyalkylénés ;
• les esters d'acides gras du polyéthylèneglycol ;
• les alkyl(C₆-C₃₀)polyglycosides ;
• les dérivés de N-alkyl(C₆-C₃₀)glucamine ;
• les oxydes d'amines tels que les oxydes d'alkyl (C₁₀ - C₁₄) amines ou les oxydes de N-acylaminopropylmorpholine ;
• les copolymères d'oxyde d'éthylène et de propylène ;
• leurs mélanges.

7. Composition selon l'une quelconque des revendications précédentes, **caractérisée en ce que** le tensioactif anionique est choisi parmi :
• les alkyl(C₆-C₃₀)sulfates, les alkyl(C₆-C₃₀)éthersulfates, les alkyl(C₆-C₃₀)amido-éthersulfates, les alkylarylpolyéthersulfates, les monoglycérides sulfates ;
• les alkyl(C₆-C₃₀)sulfonates, les alkyl(C₆-C₃₀)amidesulfonates, les atkyl(C₆-C₃₀)aryl-sulfonates, les α-oléfine-sulfonates, les paraffine-sulfonates ;
• les alkyl(C₆-C₃₀)phosphates ;
• les alkyl(C₆-C₃₀)sulfosuccinates, les alkyl(C₆-C₃₀) éthersulfosuccinates, les alkyl(C₆-C₃₀) amidesulfosuccinates ;
• les alkyl(C₆-C₃₀)sulfoacétates ;
• les acyl(C₆-C₂₄)sarcosinates ;
• les acyl(C₆-C₂₄)glutamates ;
• les éthers d'alkyl(C₆-C₃₀)polyglycosides carboxyliques; les alkyl(C₆-C₃₀) polyglycoside sulfosuccinates ;
• les alkyl(C₆-C₃₀)sulfosuccinamates ;
• les acyl(C₆-C₂₄)iséthionates ;
• les N-acyl(C₆-C₂₄)taurates ;
• les sels d'acides gras en C₆-C₃₀ ;
• les acyl(C₈-C₂₀)-lactylates ;
• les sels d'acides d'alkyl(C₆-C₃₀)-D galactoside uroniques ;
• les sels d'acides alkyl(C₆-C₃₀)éther carboxyliques polyoxyalkylénés, d'alkyl(C₆-C₃₀)aryl éther carboxyliques polyoxyalkylénés, d'alkyl(C₆-C₃₀)amido éther carboxyliques polyoxyalkylénés ;
• et leurs mélanges.

8. Composition selon l'une quelconque des revendications précédentes, **caractérisée en ce que** la teneur totale en tensioactif(s) non ionique(s), anionique(s) et leurs mélanges, représente de 0,1 % à 40 % en poids par rapport au poids de la composition colorante.

9. Composition selon l'une quelconque des revendications précédentes, **caractérisée en ce que** la composition comprend au moins un corps gras différent de l'alcool gras.

10. Composition selon la revendication précédente, **caractérisée en ce que** le corps gras est choisi parmi les amides d'acide gras non oxyalkylénés non glycérolés, les mono- et poly- esters d'acides carboxyliques, les huiles minérales, les huiles végétales, ou leurs mélanges.

11. Composition selon l'une quelconque des revendications précédentes, **caractérisée en ce que** le polymère associatif non ionique est choisi parmi les polymères suivants, seuls ou en mélanges :
- **(1)** les celluloses modifiées par des groupements comportant au moins une chaîne grasse ;
- **(2)** les hydroxypropylguars modifiés par des groupements comportant au moins une chaîne grasse ;
- **(3)** les copolymères de vinyl pyrrolidone et de monomères hydrophobes à chaîne grasse ;
- **(4)** les copolymères de méthacrylates ou d'acrylates d'alkyles en C₁-C₆ et de monomères amphiphiles comportant au moins une chaîne grasse ;
- **(5)** les copolymères de méthacrylates ou d'acrylates hydrophiles et de monomères hydrophobes comportant au moins une chaîne grasse ;
- **(6)** les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques ;
- **(7)** les polymères à squelette aminoplaste éther possédant au moins une chaîne grasse.

12. Composition selon l'une quelconque des revendications précédentes, **caractérisée en ce que** la teneur en polymère associatif non ionique représente de 0,01 à 5 % en poids par rapport au poids de la composition colorante.

13. Composition selon l'une quelconque des revendications précédentes, **caractérisée en ce que** la teneur en colorant direct représente de 0,0005 à 15 % en poids par rapport au poids de la composition colorante.

14. Composition selon l'une quelconque des revendications précédentes, **caractérisée en ce que** la composition comprend au moins un agent alcalinisant.

15. Composition selon la revendication précédente, **caractérisée en ce que** l'agent alcalinisant est choisi parmi l'ammoniaque, les alcanolamines, et les associations d'alcanolamines en C₂-C₁₀ avec des silicates de métaux alcalins ou alcali no-terreux.

16. Composition selon l'une quelconque des revendications précédentes, **caractérisée en ce qu'**elle comprend au moins un polymère substantif cationique ou amphotère.

17. Composition selon la revendication précédente, **caractérisée en ce que** a teneur en polymère substantif cationique ou amphotère représente de 0,01 % à 10 % en poids par rapport au poids de la composition colorante.

18. Composition selon l'une quelconque des revendications précédentes **caractérisée en ce qu'**elle comprend au moins un agent oxydant.

19. Procédé de coloration de fibres kératiniques, dans lequel on applique sur lesdites fibres, sèches ou humides, une composition colorante selon l'une quelconque des revendications 1 à 18.

20. Procédé de coloration de fibres kératiniques dans lequel on applique sur lesdites fibres, sèches ou humides, une composition colorante selon l'une quelconque des revendications 1 à 17, en présence d'une composition oxydante, appliquée simultanément ou successivement sans rinçage intermédiaire à la composition colorante, on laisse poser et on rince les fibres.

21. Procédé de coloration de fibres kératiniques dans lequel on applique sur lesdites fibres, sèches ou humides, une composition colorante selon l'une quelconque des revendications 1 à 17, en présence d'une composition oxydante, mélangée à la composition colorante avant l'application, on laisse poser et on rince les fibres.

22. Dispositif à plusieurs compartiments utilisable pour la coloration de fibres kératiniques, comprenant un premier compartiment renfermant une composition selon l'une quelconque des revendications 1 à 17 et comprenant un second compartiment renfermant une composition oxydante.

## Patentansprüche

1. Farbmittelzusammensetzung, die in einem zum Färben von Keratinfasern geeigneten Medium enthält:
• mindestens einen Direktfarbstoff;
• mindestens einen Fettalkohol;
• mindestens einen Ester einer Fettsäure und eines C₁₋₁₀-Alkohols, der ein Monoester einer linearen oder verzweigten, gesättigten oder ungesättigten Carbonsäure mit 8 bis 30 Kohlenstoffatomen und eines linearen oder verzweigten, gesättigten oder ungesättigten Monohydroxyalkohols mit 1 bis 10 Kohlenstoffatomen ist;
• mindestens einen nichtionischen oder anionischen grenzflächenaktiven Stoff oder deren Gemische;
• mindestens ein nichtionisches assoziatives Polymer;
• wobei der Wassergehalt mindestens 55 Gew.-%, bezogen auf das Gewicht der Farbmittelzusammensetzung, beträgt.

2. Zusammensetzung nach dem vorhergehenden Anspruch, **dadurch gekennzeichnet, dass** der Wassergehalt mindestens 60 Gew.-%, bezogen auf das Gewicht der Farbmittelzusammensetzung, ausmacht.

3. Zusammensetzung nach einem der vorhergehenden Ansprüche, **dadurch gekennzeichnet, dass** der Mengenanteil des Fettalkohols 0,1 bis 30 Gew.-%, bezogen auf das Gewicht der Farbmittelzusammensetzung, ausmacht.

4. Zusammensetzung nach dem vorhergehenden Anspruch, **dadurch gekennzeichnet, dass** der Ester unter den Monoestern von Ölsäure, Laurinsäure, Palmitinsäure, Myristinsäure, Behensäure, Stearinsäure, Linolsäure, Linolensäure, Caprinsäure, Arachidonsäure und von Methanol, Ethanol, Propanol, Isopropanol, Ethylenglycol, Glycerin, Octanol, Decanol sowie deren Gemischen ausgewählt ist.

5. Zusammensetzung nach einem der vorhergehenden Ansprüche, **dadurch gekennzeichnet, dass** der Mengenanteil des Esters einer Fettsäure und eines C₁₋₁₀-Alkohols im Bereich von 0,1 bis 30 Gew.-%, bezogen auf das Gewicht der Farbmittelzusammensetzung, liegt.

6. Zusammensetzung nach einem der vorhergehenden Ansprüche, **dadurch gekennzeichnet, dass** der nichtionische grenzflächenaktive Stoff ausgewählt ist unter:
• akoxylierten oder mit Glycerin veretherten Fettalkoholen;
• alkoxylierten Alkylphenolen, deren Alkylkette 8 bis 18 Kohlenstoffatome aufweist;
• alkoxylierten oder mit Glycerin veretherten Fettamiden;
• alkoxylierten pflanzlichen Ölen;
• Sorbitanfettsäureestern, die gegebenenfalls alkoxyliert sind;
• Saccharosefettsäureestern, die gegebenenfalls alkoxyliert sind;
• Polyethylenglycolfettsäureestern;
• Alkyl(C₆₋₃₀)polyglycosiden;
• N-Alkyl(C₆₋₃₀)glucaminderivaten;
• Aminoxiden, wie Alkyl(C₁₀₋₁₄)aminoxiden oder N-Acylaminopropylmorpholinoxiden;
• Copolymeren von Ethylenoxid und Propylenoxid;
• deren Gemischen.

7. Zusammensetzung nach einem der vorhergehenden Ansprüche,
**dadurch gekennzeichnet, dass** der anionische grenzflächenaktive Stoff ausgewählt ist unter:
• Alkyl(C₆₋₃₀)sulfaten, Alkyl(C₆₋₃₀)ethersulfaten, Alkyl(C₆₋₃₀)-amidoethersulfaten, Alkylarylpolyethersulfaten, Monoglyceridsulfaten;
• Alkyl(C₆₋₃₀)sulfonaten, Alkyl(C₆₋₃₀)amidsulfonaten, Alkyl(C₆₋₃₀)-arylsulfonaten, α-Olefinsulfonaten, Paraffinsulfonaten;
• Alkyl(C₆₋₃₀)phosphaten;
• Alkyl(C₆₋₃₀)sulfosuccinaten, Alkyl(C₆₋₃₀)ethersulfosuccinaten, Alkyl(C₆₋₃₀)amidosulfosuccinaten;
• Alkyl(C₆₋₃₀)sulfoacetaten;
• Acyl(C6-24)sarcosinaten;
• Acyl(C₆₋₂₄)glutamaten;
• Alkyl(C₆₋₃₀)polyglycosidethercarbonsäuren; Alkyl(C₆₋₃₀)poly-glycosidsulfosuccinaten;
• Alkyl(C₆₋₃₀)sulfosuccinamaten;
• Acyl(C₆₋₂₄)isethionaten;
• N-Acyl(C₆₋₂₄)tauraten;
• C₆₋₃₀-Fettsäuresalzen;
• Acyl(C₈₋₂₀)lactylaten;
• Salzen von Alkyl(C₆₋₃₀)-D-galactosiduronsäuren;
• Salzen von polyalkoxylierten Alkyl(C₆₋₃₀)ethercarbonsäuren, Salzen von polyalkoxylierten Alkyl(C₆₋₃₀)arylethercarbonsäuren, Salzen von polyalkoxylierten Alkyl(C₆₋₃₀)amidoethercarbonsäuren;
• und deren Gemischen.

8. Zusammensetzung nach einem der vorhergehenden Ansprüche, **dadurch gekennzeichnet, dass** die Gesamtmenge des (der) anionischen, nichtionischen grenzflächenaktiven Stoffe(s) 0,1 bis 40 Gew.-%, bezogen auf das Gewicht der Farbmittelzusammensetzung, ausmacht.

9. Zusammensetzung nach einem der vorhergehenden Ansprüche, **dadurch gekennzeichnet, dass** die Zusammensetzung mindestens eine Fettsubstanz enthält, die von dem Fettalkohol verschieden ist.

10. Zusammensetzung nach dem vorhergehenden Anspruch, **dadurch gekennzeichnet, dass** die Fettsubstanz unter den nicht alkoxylierten, nicht mit Glycerin veretherten Fettsäureamiden, Mono- und Polyestern von Carbonsäuren, Mineralölen, pflanzlichen Ölen oder deren Gemischen ausgewählt ist.

11. Zusammensetzung nach einem der vorhergehenden Ansprüche, **dadurch gekennzeichnet, dass** das nichtionische assoziative Polymer einzeln oder in Form von Gemischen unter den folgenden Polymeren ausgewählt ist:
- (1) Cellulosen, die mit Gruppen modifiziert sind, die mindestens eine Fettkette enthalten;
- (2) Hydroxypropylguarverbindungen, die mit Gruppen modifiziert sind, die mindestens eine Fettkette enthalten;
- (3) Copolymeren von Vinylpyrrolidon und hydrophoben Monomeren mit Fettkette;
- (4) Copolymeren von C₁₋₆-Alkylmethacrylaten oder C₁₋₆-Alkylacrylaten und amphiphilen Monomeren, die mindestens eine Fettkette enthalten;
- (5) Copolymeren von hydrophilen Methacrylaten oder Acrylaten und hydrophoben Monomeren, die mindestens eine Fettkette enthalten;
- (6) Polyurethanpolyethern, die in ihrer Kette hydrophile Sequenzen, bei denen es sich meistens um polyethoxylierte Sequenzen handelt, und gleichzeitig hydrophobe Sequenzen enthalten, die aliphatische Ketten alleine und/oder cycloaliphatische und/oder aromatische Ketten sein können;
- (7) Polymeren mit Aminoplastether-Grundgerüst, die mindestens eine Fettkette enthalten.

12. Zusammensetzung nach einem der vorhergehenden Ansprüche, **dadurch gekennzeichnet, dass** der Mengenanteil des nichtionische assoziativen Polymers 0,01 bis 5 Gew.-%, bezogen auf das Gewicht der Farbmittelzusammensetzung, ausmacht.

13. Zusammensetzung nach einem der vorhergehenden Ansprüche, **dadurch gekennzeichnet, dass** der Mengenanteil des Direktfarbstoffes 0,0005 bis 15 Gew.-%, bezogen auf das Gewicht der Farbmittelzusammensetzung, ausmacht.

14. Zusammensetzung nach einem der vorhergehenden Ansprüche, **dadurch gekennzeichnet, dass** die Zusammensetzung mindestens ein Alkalisierungsmittel enthält.

15. Zusammensetzung nach dem vorhergehenden Anspruch, **dadurch gekennzeichnet, dass** das Alkalisierungsmittel unter Ammoniak, Alkanolaminen und Kombinationen von C₂₋₁₀-Alkanolaminen und Silicaten von Alkalimetallen oder Erdalkalimetallen ausgewählt ist.

16. Zusammensetzung nach einem der vorhergehenden Ansprüche, **dadurch gekennzeichnet, dass** sie mindestens ein kationisches oder amphoteres, substantives Polymer enthält.

17. Zusammensetzung nach dem vorhergehenden Anspruch, **dadurch gekennzeichnet, dass** der Mengenanteil des kationischen oder amphoteren substantiven Polymers 0,01 bis 10 Gew.-%, bezogen auf das Gewicht der Farbmittelzusammensetzung, ausmacht.

18. Zusammensetzung nach einem der vorhergehenden Ansprüche, **dadurch gekennzeichnet, dass** sie mindestens ein Oxidationsmittel enthält.

19. Verfahren zum Färben von Keratinfasern, bei dem auf die trockenen oder feuchten Fasern eine Farbmittelzusammensetzung nach einem der Ansprüche 1 bis 18 aufgetragen wird.

20. Verfahren zum Färben von Keratinfasern, bei dem auf die trockenen oder feuchten Fasern eine Farbmittelzusammensetzung nach einem der Ansprüche 1 bis 17 in Gegenwart einer oxidierenden Zusammensetzung aufgebracht wird, die gleichzeitig mit oder ohne zwischenzeitliches Spülen nach der Farbmittelzusammensetzung aufgetragen wird, einwirken gelassen wird und die Fasern gespült werden.

21. Verfahren zum Färben von Keratinfasern, bei dem auf die trockenen oder feuchten Fasern eine Farbmittelzusammensetzung nach einem der Ansprüche 1 bis 17 in Gegenwart einer oxidierenden Zusammensetzung aufgebracht wird, die vor der Anwendung mit der Farbmittelzusammensetzung vermischt wird, einwirken gelassen wird und die Fasern gespült werden.

22. Vorrichtung mit mehreren Abteilungen, die zum Färben von Keratinfasern verwendbar ist, die eine erste Abteilung aufweist, die eine Zusammensetzung nach einem der Ansprüche 1 bis 17 enthält, und die eine zweite Abteilung aufweist, die eine oxidierende Zusammensetzung enthält.

## Claims

1. Dye composition comprising, in a medium suitable for dyeing keratin fibres:
• at least one direct dye;
• at least one fatty alcohol;
• at least one fatty acid ester of a C₁-C₁₀ alcohol which is a monoester of a linear or branched, saturated or unsaturated C₈-C₃₀ carboxylic acid and of a linear or branched, saturated or unsaturated C₁-C₁₀ monohydroxylated alcohol;
• at least one non-ionic or anionic surfactant, or mixtures thereof;
• at least one non-ionic associative polymer;
• the water content being at least 55% by weight relative to the weight of the dye composition.

2. Composition according to the preceding claim, **characterized in that** the water content represents at least 60% by weight relative to the weight of said dye composition.

3. Composition according to either one of the preceding claims, **characterized in that** the fatty alcohol content represents from 0.1% to 30% by weight relative to the weight of the dye composition.

4. Composition according to the preceding claim, **characterized in that** the ester is chosen from the monoesters of oleic acid, lauric acid, palmitic acid, myristic acid, behenic acid, stearic acid, linoleic acid, linolenic acid, capric acid or arachidonic acid, and of methanol, ethanol, propanol, isopropanol, ethylene glycol, glycerol, octanol or decanol, and also mixtures thereof.

5. Composition according to any one of the preceding claims, **characterized in that** the content of fatty acid ester of a C₁-C₁₀ alcohol is between 0.1% and 30% by weight relative to the weight of the dye composition.

6. Composition according to any one of the preceding claims, **characterized in that** the non-ionic surfactant is chosen from:
• oxyalkylenated or glycerolated fatty alcohols;
• oxyalkylenated alkylphenols in which the alkyl chain is of C₈-C₁₈;
• oxyalkylenated or glycerolated fatty amides;
• oxyalkylenated plant oils;
• optionally oxyalkylenated fatty acid esters of sorbitan;
• optionally oxyalkylenated fatty acid esters of sucrose;
• fatty acid esters of polyethylene glycol;
• (C₆-C₃₀)alkyl polyglycosides;
• N-(C₆-C₃₀)alkylglucamine derivatives;
• amine oxides such as (C₁₀-C₁₄)alkylamine oxides or N-acylaminopropylmorpholine oxides;
• copolymers of ethylene oxide and of propylene oxide;
• mixtures thereof.

7. Composition according to any one of the preceding claims, **characterized in that** the anionic surfactant is chosen from:
• (C₆-C₃₀)alkyl sulphates, (C₆-C₃₀)alkyl ether sulphates, (C₆-C₃₀)alkylamido ether sulphates, alkylaryl polyether sulphates, monoglyceride sulphates;
• (C₆-C₃₀)alkyl sulphonates, (C₆-C₃₀)alkylamide sulphonates, (C₆-C₃₀)alkylaryl sulphonates, α-olefin sulphonates, paraffin sulphonates;
• (C₆-C₃₀)alkyl phosphates;
• (C₆-C₃₀)alkyl sulphosuccinates, (C₆-C₃₀)alkyl ether sulphosuccinates, (C₆-C₃₀)alkylamide sulphosuccinates;
• (C₆-C₃₀)alkyl sulphoacetates;
• (C₆-C₂₄)acyl sarcosinates;
• (C₆-C₂₄)acyl glutamates;
• (C₆-C₃₀)alkylpolyglycoside carboxylic ethers; (C₆-C₃₀)alkylpolyglycoside sulphosuccinates;
• (C₆-C₃₀)alkyl sulphosuccinamates;
• (C₆-C₂₄)acyl isethionates;
• N-(C₆-C₂₄)acyl taurates;
• C₆-C₃₀ fatty acid salts;
• (C₈-C₂₀)acyl lactylates;
• (C₆-C₃₀)alkyl-D-galactoside uronic acid salts;
• polyoxyalkylenated (C₆-C₃₀)alkyl ether carboxylic acid salts, polyoxyalkylenated (C₆-C₃₀)alkylaryl ether carboxylic acid salts, polyoxyalkylenated (C₆-C₃₀)alkylamido ether carboxylic acid salts;
• and mixtures thereof.

8. Composition according to any one of the preceding claims, **characterized in that** the total content of non-ionic surfactant(s), anionic surfactant(s) and mixtures thereof represents from 0.1% to 40% by weight relative to the weight of the dye composition.

9. Composition according to any one of the preceding claims, **characterized in that** the composition comprises at least one fatty substance other than the fatty alcohol.

10. Composition according to the preceding claim, **characterized in that** the fatty substance is chosen from non-oxyalkylenated, non-glycerolated fatty acid amides, carboxylic acid monoesters and polyesters, mineral oils and plant oils, or mixtures thereof.

11. Composition according to any one of the preceding claims, **characterized in that** the non-ionic associative polymer is chosen from the following polymers, alone or as mixtures:
- **(1)** celluloses modified with groups comprising at least one fatty chain;
- **(2)** hydroxypropyl guars modified with groups comprising at least one fatty chain;
- **(3)** copolymers of vinylpyrrolidone and of hydrophobic monomers comprising a fatty chain;
- **(4)** copolymers of C₁-C₆ alkyl methacrylates or acrylates and of amphiphilic monomers comprising at least one fatty chain;
- **(5)** copolymers of hydrophilic methacrylates or acrylates and of hydrophobic monomers comprising at least one fatty chain;
- **(6)** polyether polyurethanes comprising, in their chain, both hydrophilic blocks that are most commonly polyoxyethylenated in nature and hydrophobic blocks which may be aliphatic chains alone and/or cycloaliphatic and/or aromatic chains;
- **(7)** polymers with an aminoplast-ether backbone having at least one fatty chain.

12. Composition according to any one of the preceding claims, **characterized in that** the content of non-ionic associative polymer represents from 0.01% to 5% by weight relative to the weight of the dye composition.

13. Composition according to any one of the preceding claims, **characterized in that** the content of direct dye represents from 0.0005% to 15% by weight relative to the weight of the dye composition.

14. Composition according to any one of the preceding claims, **characterized in that** the composition comprises at least one basifying agent.

15. Composition according to the preceding claim, **characterized in that** the basifying agent is chosen from aqueous ammonia, alkanolamines and combinations of C₂-C₁₀ alkanolamines with alkali metal or alkaline-earth metal silicates.

16. Composition according to any one of the preceding claims, **characterized in that** it comprises at least one cationic or amphoteric substantive polymer.

17. Composition according to the preceding claim, **characterized in that** the content of cationic or amphoteric substantive polymer represents from 0.01% to 10% by weight relative to the weight of the dye composition.

18. Composition according to any one of the preceding claims, **characterized in that** it comprises at least one oxidizing agent.

19. Process for dyeing keratin fibres, in which a dye composition according to any one of Claims 1 to 18 is applied to said fibres, which may be dry or wet.

20. Process for dyeing keratin fibres, in which a dye composition according to any one of Claims 1 to 17 is applied to said fibres, which may be dry or wet, in the presence of an oxidizing composition, which is applied simultaneously with or successively to the dye composition without intermediate rinsing, the mixture is left on the fibres, and the fibres are rinsed.

21. Process for dyeing keratin fibres, in which a dye composition according to any one of Claims 1 to 17 is applied to said fibres, which may be dry or wet, in the presence of an oxidizing composition, which is mixed with the dye composition before application, the mixture is left on the fibres, and the fibres are rinsed.

22. Multicompartment device that may be used for dyeing keratin fibres, comprising a first compartment containing a composition according to any one of Claims 1 to 20 and comprising a second compartment containing an oxidizing composition.
